# EUROPEAN PATENT SPECIFICATION

(11) **EP 0 942 729 B1**
(45) Date of publication and mention of the grant of the patent: **01.09.2004**
(21) Application number: 97952020.2
(22) Date of filing: 25.11.1997
(51) Int. Cl.: A61K 31/535, A61K 31/445

(54) **USE OF NK-1 RECEPTOR ANTAGONISTS FOR TREATING STRESS DISORDERS**
DIE VERWENDUNG VON NK-1 REZEPTOR ANTAGONISTEN FÜR DIE BEHANDLUNGEN VON STRESS STÖRUNGEN
UTILISATION D'ANTAGONISTES DU RECEPTEUR NK-1 DANS LE TRAITEMENT DES ETATS DE STRESS

(30) Priority: 02.12.1996 GB 9625051; 24.01.1997 GB 9701459; 27.06.1997 GB 9713715; 04.08.1997 GB 9716482; 07.10.1997 GB 9721171
(43) Date of publication of application: 22.09.1999
(73) Proprietor: MERCK SHARP & DOHME LTD., Hoddesdon, Hertfordshire EN11 9BU (GB)
(72) Inventor: BAKER, Raymond, North Star Avenue Swindon SN2 1UH (GB); CURTIS, Neil, Roy, Harlow Essex CM20 2QR (GB); ELLIOTT, Jason, Matthew, Harlow Essex CM20 2QR (GB); HARRISON, Timothy, Harlow Essex CM20 2QR (GB); HOLLINGWORTH, Gregory, John, Harlow Essex CM20 2QR (GB); JACKSON, Philip, Stephen, Harlow Essex CM20 2QR (GB); KULAGOWSKI, Janusz, Jozef, Harlow Essex CM20 2QR (GB); RUPNIAK, Nadia, Melanie, Harlow Essex CM20 2QR (GB); SEWARD, Eileen, Mary, Harlow Essex CM20 2QR (GB); SWAIN, Christopher, John, Harlow Essex CM20 2QR (GB); WILLIAMS, Brian, John, Harlow Essex CM20 2QR (GB)
(74) Representative: Horgan, James Michael Frederic
(86) International application number: PCT/EP1997/006684
(87) International publication number: WO 1998/024440

(56) References cited:
- EP-A- 0 528 495
- EP-A- 0 577 394
- WO-A-95/08549
- WO-A-95/18124
- WO-A-96/05181
- DE-A- 4 002 840
- US-A- 5 496 833

## Description

This invention relates to the treatment or prevention of certain stress disorders by the administration of a specific class of NK-1 receptor antagonists.

Stressors have been described as physiological or psychological perturbances which disrupt an individual's homeostatic balance, and the stress-response is the set of neural and endocrine adaptations that come into effect to reestablish homeostasis (R.M. Sapolsky, "Stress, the Aging Brain, and the Mechanisms of Neuron Death", the MIT Press, Cambridge, Massachusetts, 1992). The stress-response is surprisingly convergent in that stressors as diverse as injury, starvation, temperature extremes and anxiety will provoke similar stress-responses. With prolonged stress, however, the stress-response can become as damaging as the stressor itself. Thus, chronic overactivation of the stress-response, whether due to too much stress or due to an inability to "turn-off" the stress-response, may lead to stress-related conditions including myopathy, steroid diabetes, hypertension, peptic ulcers, reproductive impairment, psychogenic dwarfism and immunosuppression.

Stress disorders may result from over-exposure to a stressor, an adjustment reaction (for example, to serious illness or breavement) or in response to an exceptionally stressful trauma.

Apart from counselling, treatment of stress disorders may include the use of anxiolytic drugs such as benzodiazepines or tricyclic antidepressants, or hypnotic drugs. Potent benzodiazepines should not be prescribed for more than 3 or 4 weeks, however, due to the risks associated with drug dependency. Benzodiazepines are also associated with a number of side-effects including increased hostility and irritability, vivid or disturbing dreams, weight gain, skin rash, nausea, headache, impairment of sexual function, vertigo, and lightheadedness. Difficulties may also arise with the use of tricyclic antidepressants (TCAs), in particular, due to their anticholinergic side-effects which are particularly troublesome in patients with prostatic enlargement or glaucoma. Other side-effects of tricyclic antidepressants include dry mouth, tachycardia, difficulty in visual accomodation, constipation, urinary retention, sexual dysfunction, cognitive impairment, postural hypotension, and weight gain.

Selective serotonin reuptake inhibitors (SSRIs) may also be prescribed for the treatment of stress disorders but these too are not without their side-effects including nausea, diarrhoea, dry mouth, reduced appetite, dyspepsia, vomiting, headache, nervousness, insomnia, anxiety, tremor, dizziness, fatigue, decreased libido, pharyngitis, dyspnoea, skin rash and sexual dysfunction.

When TCAs or SSRIs are prescribed, there is a delay of usually two to three weeks before a therapeutic effect is observed. This period of delay may be particularly difficult for a patient suffering from a stress disorder.

Neurokinin 1 (NK-1; substance P) receptor antagonists are being developed for the treatment of a number of physiological disorders associated with an excess or imbalance of tachykinins, and in particular substance P: Examples of conditions in which substance P has been implicated include disorders of the central nervous system such as anxiety, depression and psychosis (see, for instance, International (PCT) patent specification Nos. WO 95/16679, WO 95/18124 and WO 95/23798).

On the other hand, European Patent Specification No. 0 286 928 describes inhibitors of the enzyme prolyl-endopeptidase, which enzyme degrades neuropeptides such as substance P, the enzyme inhibitors having an antipsychotic, anxiolytic and antidepressant action. Thus, degrading substance P or reducing the action of substance P in some other way (e.g. antagonism at its preferred NK-1 receptor) might be expected to be detrimental to the treatment of stress disorders.

Furthermore, European Patent Specification No. 0 490 379 describes diamino derivatives, related to the C-terminus of substance P, for use in the treatment of stress-induced conditions. These diamino derivatives are agonists of substance P. Thus, use of an NK-1 receptor antagonist (substance P being the preferred ligand for the NK-1 receptor in humans) might be expected to be detrimental to the treatment of stress disorders.

More recently, International (PCT) patent specification No. WO 96/24353 (published 15th August 1996) suggests that a more efficacious and safe treatment of psychiatric disorders would be achieved using a combination of a tachykinin antagonist and a serotonin agonist or selective serotonin reuptake inhibitor (SSRI). However, such a regimen would not be free of side-effects due to the serotonin agonist or SSRI.

NK-1 receptor antagonists are described in published European Patent Specification Nos. 0 360 390, 0 394 989, 0 429 366, 0 443 132, 0 482 539, 0 512 901, 0 512 902, 0 514 273, 0 514 275, 0 517 589, 0 520 555, 0 522 808, 0 528 495, 0 532 456, 0 533 280, 0 536 817, 0 545 478, 0 577 394, 0 590 152, 0 599 538, 0 610 793, 0 634 402, 0 686 629, 0 693 489, 0 694 535, 0 699 655, 0 699 674, 0 707 006, 0 708 101, 0 714 891, 0 723 959, 0 733 632 and 0 776 893; and in International Patent Specification Nos. 90/05525, 90/05729, 91/09844, 91/18899, 92/01688, 92/06079, 92/12151, 92/15585, 92/17449, 92/20661, 92/20676, 92/21677, 93/00330, 93/00331, 93/01159, 93/01165, 93/01169, 93/01170, 93/06099, 93/09116, 93/10073, 93/14113, 93/18023, 93/19064, 93/21155, 9321181, 93/23380, 93/24465, 94/01402, 94/02461, 94/03429, 94/03445, 94/04494, 94/04496, 94/05625, 94/07843, 94/10165, 94/10167, 94/10168, 94/10170, 94/11368, 94/13639, 94/13663, 94/14767, 94/15903, 94/19320, 94/19323, 94/20500, 94/26735, 94/26740, 94/29309, 95/02595, 95/04040, 95/04042, 95/06645, 95/07886, 95/07908, 95/08549, 95/11880, 95/14017, 95/15311, 95/16679, 95/17382, 95/18124, 95/18129, 95/19344, 95/20575, 95/21819, 96/22525, 95/23798, 95/26338, 95/28418, 95/30674, 95/30687, 96/05193, 96/05203, 96/06094, 96/07649, 96/10562, 96/16939, 96/18643, 96/20197, 96/21661, 96/29304, 96/29317, 96/29326, 96/29328, 96/31214, 96/32385, 96/37489, 97/01553, 97/01554, 97/03066, 97/08144, 97/14671, 97/17362, 97/18206, 97/19084, 97/19942 and 97/21702; and in British Patent Specification Nos. 2 266 529, 2 268 931, 2 269 170, 2 269 590, 2 271 774, 2 292 144, 2 293 168, 2 293 169, and 2 302 689.

In view of the short-comings of existing anti-stress therapy, there is a need for new, safe and effective treatment for stress disorders.

The present invention provides the use of a CNS penetrant NK-1 receptor antagonist in an oral, once-a-day medicament for the treatment of stress disorders. The compounds of this class advantageously exhibit a rapid onset of action and a reduced side-effect profile when compared against conventional anxiolytic agents.

In particular, the present invention provides a means for the identification of NK-1 receptor antagonists which would be effective in an oral once-a-day medicament for the treatment of stress disorders. The aforementioned patent specifications which describe NK-1 receptor antagonists provide no reliable method for the identification of such compounds.

The exceptional pharmacology of the class of NK-1 receptor antagonists of use in the present invention enables the treatment of stress disorders, without the need for concomitant therapy using benzodiazepines, tricyclic antidepressants or hypnotics, or in particular, without the need for concomitant use of a serotonin agonist or an SSRI.

Furthermore, the exceptional pharmacology of the class of NK-1 receptor antagonists of use in the present invention results in a rapid onset of action.

The present invention accordingly provides the use of a NK-1 receptor antagonist for the manufacture of a medicament for oral administration for the treatment or prevention of a stress disorder selected from acute stress disorder, adjustment disorder and acute, chronic or delayed onset post-traumatic stress disorder, without concomitant therapy with other anti-stress agents,
wherein said NK-1 receptor antagonist is CNS-penetrant as determined by its ability to inhibit NK-1 receptor agonist-induced foot-tapping in the gerbil, and is effective in the attenuation of separation-induced vocalisations by guinea-pig pups,
and wherein said NK-1 receptor antagonist is long acting as determined by its ability to inhibit foot-tapping in the gerbil with ≤ 25-fold loss of potency where the NK-1 receptor antagonist is administered 24 hours prior to NK-1 receptor agonist challenge compared with the potency when administered immediately prior to the NK-1 receptor agonist challenge, with the proviso that the ID₅₀≤10mg/kg i.v. after 24 hour pre-treatment.

There exists a patient population in whom stress disorders are inadequately treated with benzodiazepines. Furthermore, some patients may be adversely affected by the side-effects of benzodiazepines.

The present invention accordingly provides the use of a NK-1 receptor antagonist for the manufacture of a medicament for oral administration for the treatment or prevention of a stress disorder selected from acute stress disorder, adjustment disorder and acute, chronic or delayed onset post-traumatic stress disorder, without concomitant therapy with other anti-stress agents, in a patient who is non-responsive to benzodiazepines or for whom benzodiazepines are contraindicated,
wherein said NK-1 receptor antagonist is CNS-penetrant as determined by its ability to inhibit NK-1 receptor agonist-induced foot-tapping in the gerbil, and is effective in the attenuation of separation-induced vocalisations by guinea-pig pups,
and wherein said NK-1 receptor antagonist is long acting as determined by its ability to inhibit foot-tapping in the gerbil with ≤ 25-fold loss of potency where the NK-1 receptor antagonist is administered 24 hours prior to NK-1 receptor agonist challenge compared with the potency when administered immediately prior to the NK-1 receptor agonist challenge, with the proviso that the ID₅₀≤10mg/kg i.v. after 24 hour pre-treatment.

Furthermore, there exists a patient population in whom stress disorders are inadequately treated with heterocyclic antidepressants (TCAs, tetracyclics and the like) or SSRIs. Furthermore, some patients may be adversely affected by the side-effects of heterocyclic antidepressants (TCAs, tetracyclics and the like) or SSRIs.

The present invention accordingly provides the use of a NK-1 receptor antagonist for the manufacture of a medicament for oral administration for the treatment or prevention of a stress disorder selected from acute stress disorder, adjustment disorder and acute, chronic or delayed onset post-traumatic stress disorder without concomitant therapy with other anti-stress agents, in a patient who is non-responsive to heterocyclic antidepressants or SSRIs, or for whom heterocyclic antidepressants or SSRIs are contraindicated,
wherein said NK-1 receptor antagonist is CNS-penetrant as determined by its ability to inhibit NK-1 receptor agonist-induced foot-tapping in the gerbil, and is effective in the attenuation of separation-induced vocalisations by guinea-pig pups,
and wherein said NK-1 receptor antagonist is long acting as determined by its ability to inhibit foot-tapping in the gerbil with ≤ 25-fold loss of potency where the NK-1 receptor antagonist is administered 24 hours prior to NK-1 receptor agonist challenge compared with the potency when administered immediately prior to the NK-1 receptor agonist challenge, with the proviso that the ID₅₀≤10mg/kg i.v. after 24 hour pre-treatment.

As used herein, the term "stress disorders" is intended to denote acute stress disorder, adjustment disorder and acute, chronic or delayed onset post-traumatic stress disorder.

As used herein, the term "treatment" refers both to the treatment and to the prevention or prophylactic therapy of the aforementioned conditions.

Preferred NK-1 receptor antagonists for use in the present invention are selected from the classes of compounds described in European Patent Specification No. 0 577 394, and International Patent Specification Nos. 95/08549, 95/18124, 95/23798 and 96/05181, and International Patent Application No. PCT/GB97/01630. The preparation of such compounds is fully described in the aforementioned publications.

A particularly preferred NK-1 receptor antagonist of use in the present invention is:
2-(R)-(1-(R)-(3,5-bis(trifluoromethyl)phenyl)ethoxy)-3-(S)-(4-fluorophenyl)-4-(3-(5-oxo-1H,4H-1,2,4-triazolo)methyl)morpholine;
or a pharmaceutically acceptable salt thereof.

Full descriptions of the preparation of the NK-1 receptor antagonists which may be employed in the present invention may be found in the references cited herein.

Suitable pharmaceutically acceptable salts of the NK-1 receptor antagonists of use in the present invention include acid addition salts which may, for example, be formed by mixing a solution of the compound with a solution of a pharmaceutically acceptable non-toxic acid such as hydrochloric acid, fumaric acid, maleic acid, succinic acid, acetic acid, citric acid, tartaric acid, carbonic acid, phosphoric acid or sulphuric acid. Salts of amine groups may also comprise the quaternary ammonium salts in which the amino nitrogen atom carries an alkyl, alkenyl, alkynyl or .. aralkyl group. Where the compound carries an acidic group, for example a carboxylic acid group, the present invention also contemplates salts thereof, preferably non-toxic pharmaceutically acceptable salts thereof, such as the sodium, potassium and calcium salts thereof.

Preferably the compositions containing an NK-1 receptor antagonist of use according to the present invention are in unit dosage forms such as tablets, pills, capsules, wafers and the like. Additionally, the NK-1 receptor antagonists of use according to the present invention may be presented as granules or powders for extemporaneous formulation as volume defined solutions or suspensions. Alternatively, the NK-1 receptor antagonists of use according to the present invention may be presented in ready-prepared volume defined solutions or suspensions. Preferred forms are tablets and capsules.

For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing from 0.1 to about 500 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

The liquid forms in which the novel compositions of the present invention may be incorporated for administration orally include aqueous solutions, suitably flavoured syrups, aqueous or oil suspensions, and flavoured emulsions with edible oils such as cottonseed oil, sesame oil, coconut oil, peanut oil or soybean oil, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, polyvinyl-pyrrolidone or gelatin.

Compositions of the present invention may also be administered via the buccal cavity using conventional technology, for example, absorption wafers.

Compositions in the form of tablets, pills, capsules or wafers for oral administration are particularly preferred.

A minimum dosage level for the NK-1 receptor antagonist is about 1mg per day, preferably about 5mg per day and especially about 10mg per day. A maximum dosage level for the NK-1 receptor antagonist is about 1500mg per day, preferably about 1000mg per day and especially about 500mg per day. The compounds are administered once a day.

It will be appreciated that the amount of the NK-1 receptor antagonist required for use in the treatment or prevention of stress disorders will vary not only with the particular compounds or compositions selected but also with the route of administration, the nature of the condition being treated, and the age and condition of the patient, and will ultimately be at the discretion of the patient's physician or pharmacist.

Particularly preferred NK-1 receptor antagonists of use in the present invention are compounds which are potent NK-1 receptor antagonists, i.e. compounds with an NK-1 receptor affinity (IC₅₀) of less than 10nM, favourably less than 2nM and preferably less than 1nM.

The class of orally active, long acting, CNS-penetrant NK-1 receptor antagonists of use in the present invention is identified using a combination of the following assays:

### ASSAY 1: NK-1 Receptor binding

NK-1 receptor binding assays are performed in intact Chinese hamster ovary (CHO) cells expressing the human NK-1 receptor using a modification of the assay conditions described by Cascieri *et al*, *J. Pharmacol*. *Exp. Ther*., 1992, **42,** 458. Typically, the receptor is expressed at a level of 3x10⁵ receptors per cell. Cells are grown in monolayer culture, detached from the plate with enzyme-free dissociation solution (Speciality Media Inc.), and washed prior to use in the assay. ¹²⁵I-Tyr⁸-substance P (0.1nM, 2000Ci/mmol; New England Nuclear) is incubated in the presence or absence of test compounds (dissolved in 5µl dimethylsulphoxide, DMSO) with 5x10⁴ CHO cells. Ligand binding is performed in 0.25ml of 50mM Tris-HCl, pH7.5, containing 5mM MnCl₂, 150mM NaCl, 0.02% bovine serum albumin (Sigma), 50µg/ml chymostatin (Peninsula), 0.1nM phenylmethylsulphonyl fluoride, 2µg/ml pepstatin, 2µg/ml leupeptin and 2.8µg/ml furoyl saccharine. The incubation proceeds at room temperature until equilibrium is achieved (>40 minutes) and the receptor-ligand complex is harvested by filtration over GF/C filters pre-soaked in 0.1% polyethylenimine using a Tomtek 96-well harvester. Nonspecific binding is determined using excess substance P (1µM) and represents <10% of total binding.

### ASSAY 2: Gerbil Foot-Tapping

CNS-penetrant NK-1 receptor antagonists for use in the present invention can be identified by their ability to inhibit foot tapping in gerbils induced by anxiogenic agents (such as pentagastrin) or central infusion of NK-1 receptor agonists such as GR73632, or caused by aversive stimulation such as foot shock or single housing, based on the method of Rupniak & Williams, *Eur*. *J*. *Pharmacol*., 1994, **265,** 179.

Male or female Mongolian gerbils (35-70g) are anaesthetised by inhalation of an isoflurane/oxygen mixture to permit exposure of the jugular vein in order to permit administration of test compounds or vehicle in an injection volume of 5ml/kg i.v. Alternatively, test compounds may be administered orally or by subcutaneous or intraperitoneal routes. A skin incision is then made in the midline of the scalp to expose the skull. An anxiogenic agent (e.g. pentagastrin) or a selective NK-1 receptor agonist (e.g. GR73632 (d Ala[L-Pro⁹,Me-Leu¹⁰]-substance P-(7-11)) is infused directly into the cerebral ventricles (e.g. 3pmol in 5µl i.c.v., depending on test substance) by vertical insertion of a cuffed 27 gauge needle to a depth of 4.5mm below bregma. The scalp incision is closed and the animal allowed to recover from anaesthesia in a clear perspex observation box (25cm x 20cm x 20cm). The duration and/or intensity of hind foot tapping is then recorded continuously for approximately 5 minutes. Alternatively, the ability of test compounds to inhibit foot tapping evoked by aversive stimulation, such as foot shock or single housing, may be studied using a similar method of quantification.

### ASSAY 3: Ferret Emesis

Individually housed male ferrets (1.0 -2.5 kg) are dosed orally by gavage with test compound. Ten minutes later they are fed with approximately 100g of tinned cat food. At 60 minutes following oral dosing, cisplatin (10mg/kg) is given i.v. *via* a jugular vein catheter inserted under a brief period of halothane anaesthesia. The catheter is then removed, the jugular vein ligated and the skin incision closed. The ferrets recover rapidly from the anaesthetic and are mobile within 10-20 minutes. The animals are observed continuously during recovery from the anaesthetic and for 4 hours following the cisplatin injection, after which time the animals are killed humanely. The numbers of retches and vomits occurring during the 4 hours after cisplatin administration are recorded by trained observers.

### ASSAY 4: Separation-Induced Vocalisation

Male and female guinea-pigs pups are housed in family groups with their mothers and littermates throughout the study. Experiments are commenced after weaning when the pups are 2 weeks old. Before entering an experiment, the pups are screened to ensure that a vigorous vocalisation response is reproducibly elicited following maternal separation. The pups are placed individually in an observation cage (55cm x 39cm x 19cm) in a room physically isolated from the home cage for 15 minutes and the duration of vocalisation during this baseline period is recorded. Only animals which vocalise for longer than 5 minutes are employed for drug challenge studies (approximately 50% of available pups may fail to reach this criterion). On test days each pup receives an oral dose or an s.c. or i.p. injection of test compound or vehicle and is then immediately returned to the home cage with its mother and siblings for 30 to 60 minutes (or for up to 4 hours following an oral dose, dependent upon the oral pharmacokinetics of the test compound) before social isolation for 15 minutes as described above. The duration of vocalisation on drug treatment days is expressed as a percentage of the pre-treatment baseline value for each animal. The same subjects are retested once weekly for up to 6 weeks. Between 6 and 8 animals receive each test compound at each dose tested.

As used herein, the term "CNS-penetrant" refers to NK-1 receptor antagonists which are able to inhibit NK-1 receptor agonist-induced foot-tapping in the gerbil as hereinafter defined.

Essentially, hind foot-tapping in the gerbil induced by infusion of the NK-1 receptor agonist, GR73632 (d Ala[L-Pro⁹,Me-Leu¹⁰]-substance P-(7-11)), under anaesthesia, directly into the central ventricles is inhibited when a CNS-penetrant NK-1 receptor antagonist is administered intravenously immediately prior to GR73632 challenge, wherein hind foot-tapping over a period of five minutes following recovery from the anaesthesia is inhibited with an ID₅₀≤3mg/kg, and preferably with an ID₅₀≤1mg/kg.

In an alternative method, the NK-1 receptor antagonist is administered orally, 1 hour prior to GR73632 challenge, wherein the foot-tapping over a period of five minutes following recovery from anaesthesia is inhibited with an ID₅₀≤30mg/kg, and preferably with an ID₅₀≤10mg/kg.

CNS-penetrant NK-1 receptor antagonists of use in the present invention are also effective in the attenuation of separation-induced vocalisations by guinea-pig pups as hereinafter defined.

Essentially, a vocalisation response in guinea-pig pups is induced by isolation from their mothers and littermates, which response is attenuated when a CNS-penetrant NK-1 receptor antagonist is administered subcutaneously 30 minutes prior to isolation, wherein vocalisations during the first 15 minutes of isolation are attenuated with an ID₅₀≤20mg/kg, preferably with an ID₅₀≤10mg/kg, and especially with an ID₅₀≤5mg/kg.

In an alternative method, the NK-1 receptor antagonist is administered orally, 4 hours prior to isolation, wherein vocalisations during the first 15 minutes of isolation are attenuated with an ID₅₀≤20mg/kg, preferably with an ID₅₀≤10mg/kg, and especially with an ID₅₀≤5mg/kg.

A suitable selection cascade for NK₁ antagonists of use according to the present invention is as follows:
(i) Determine affinity for human NK₁ receptor in radioligand binding studies (Assay 1); select compounds with IC₅₀ ≤ 10nM, preferably IC₅₀ ≤ 2nM, especially IC₅₀ ≤ 1nM.
(ii) Determine ability of compounds to penetrate CNS by their ability to inhibit foot tapping in gerbils induced by central injection of an NK₁ agonist (Assay 2); select compounds that inhibit foot tapping with ID₅₀ ≤ 3mg/kg i.v., and preferably ID₅₀ ≤ 1mg/kg i.v. when administered immediately prior to central NK₁ agonist challenge, or ID₅₀ ≤ 30mg/kg p.o., and preferably ID₅₀ ≤ 10mg/kg p.o. 1 hour prior to challenge.
(iii) Determine central duration of action of compounds in gerbil foot tapping assay following intravenous administration 24 hours prior to central NK₁ agonist challenge; select compounds showing ≤ 25-fold loss of potency compared with ID₅₀ determined in step (ii) above with the proviso that ID₅₀ ≤ 10mg/kg i.v., and preferably ≤ 5mg/kg i.v. after 24 hour pre-treatment.
(iv) Determine oral bioavailability of compounds by pharmacokinetic analysis, activity in gerbil foot tapping assay following oral administration and/or by ability to inhibit cisplatin-induced emesis in ferrets (Assay 3); select compounds with ID₉₀ ≤ 3mg/kg p.o., and preferably ID₉₀ ≤ 1mg/kg p.o.
   Particularly preferred compounds of use in the present invention are identified using steps (i) to (iv) followed by step (v):
(v) Determine activity of compounds in assays sensitive to conventional antipsychotic drugs (inhibition of distress vocalisations in guinea-pig pups (Assay 4)). Select compounds with ID₅₀ ≤ 20mg/kg, and preferably ID₅₀ ≤ 10mg/kg.

Yet further preferred compounds of use in the present invention may be selected from those compounds which satisfy the NK-1 receptor binding criteria of step (i) which, in addition, have ≤ 5-fold shift in affinity when incubated in the presence of human serum albumin (HSA) to show non-specific protein binding.

One example of a NK-1 receptor antagonist of use in the present invention is the compound 2-(R)-(1-(R)-(3,5-bis(trifluoromethyl)phenyl)-ethoxy)-3-(S)-(4-fluorophenyl)-4-(3-(5-oxo-1H,4H-1,2,4-triazolo)methyl)-morpholine, the preparation of which is described in International Patent Specification No. WO 95/16679. In the aforementioned assays, this compound has the following activity:

| | |
|---|---|
| human NK-1 receptor binding | IC₅₀=0.1nM |
| gerbil foot-tapping (5 mins.) | ID₅₀=0.36mg/kg i.v. |
| gerbil foot-tapping (24 hrs.) | ID₅₀=0.33mg/kg i.v. |
| ferret emesis | ID₉₀<3mg/kg p.o. |
| guinea-pig vocalisation (4 hr. pre-treatment) | ID₅₀=0.73mg/kg p.o. |

The following example illustrates pharmaceutical compositions according to the invention.

### EXAMPLE 1 Tablets containing 50-300mg of NK-1 antagonist

| | Amount mg | | |
|---|---|---|---|
| NK-1 antagonist | 50.0 | 100.0 | 300.0 |
| Microcrystalline cellulose | 80.0 | 80.0 | 80.0 |
| Modified food corn starch | 80.0 | 80.0 | 80.0 |
| Lactose | 189.5 | 139.5 | 139.5 |
| Magnesium Stearate | 0.5 | 0.5 | 0.5 |

The active ingredient, cellulose, lactose and a portion of the corn starch are mixed and granulated with 10% corn starch paste. The resulting granulation is sieved, dried and blended with the remainder of the corn starch and the magnesium stearate. The resulting granulation is then compressed into tablets containing 50mg, 100mg and 300mg of the NK-1 receptor antagonist per tablet.

## Claims

1. Use of a NK-1 receptor antagonist for the manufacture of a medicament for oral administration for the treatment or prevention of a stress disorder selected from acute stress disorder, adjustment disorder and acute, chronic or delayed onset post-traumatic stress disorder, without concomitant therapy with other anti-stress agents,
wherein said NK·1 receptor antagonist is CNS-penetrant as determined by its ability to inhibit NK-1 receptor agonist-induced foot-tapping in the gerbil, and is effective in the attenuation of separation-induced vocalisations by guinea-pig pups,
and wherein said NK-1 receptor antagonist is long acting as determined by its ability to inhibit foot-tapping in the gerbil with ≤ 25-fold loss of potency where the NK-1 receptor antagonist is administered 24 hours prior to NK-1 receptor agonist challenge compared with the potency when administered immediately prior to the NK-1 receptor agonist challenge, with the proviso that the ID₅₀≤10mg/kg i.v. after 24 hour pre-treatment.

2. Use of a NK-1 receptor antagonist for the manufacture of a medicament for oral administration for the treatment or prevention of a stress disorder selected from acute stress disorder, adjustment disorder and acute, chronic or delayed onset post-traumatic stress disorder, without concomitant therapy with other anti-stress agents, in a patient who is non-responsive to benzodiazepines or for whom benzodiazepines are contraindicated,
wherein said NK-1 receptor antagonist is CNS-penetrant as determined by its ability to inhibit NK-1 receptor agonist-induced foot-tapping in the gerbil, and is effective in the attenuation of separation-induced vocalisations by guinea-pig pups,
and wherein said NK-1 receptor antagonist is long acting as determined by its ability to inhibit foot-tapping in the gerbil with ≤ 25-fold loss of potency where the NK-1 receptor antagonist is administered 24 hours prior to NK-1 receptor agonist challenge compared with the potency when administered immediately prior to the NK-1 receptor agonist challenge, with the proviso that the ID₅₀≤10mg/kg i.v. after 24 hour pre-treatment.

3. Use of a NK-1 receptor antagonist for the manufacture of a medicament for oral administration for the treatment or prevention of a stress disorder selected from acute stress disorder, adjustment disorder and acute, chronic or delayed onset post-traumatic stress disorder without concomitant therapy with other anti-stress agents, in a patient who is non-responsive to heterocyclic antidepressants or SSRIs, or for whom heterocyclic antidepressants or SSRIs are contraindicated,
wherein said NK-1 receptor antagonist is CNS-penetrant as determined by its ability to inhibit NK-1 receptor agonist-induced foot-tapping in the gerbil, and is effective in the attenuation of separation-induced vocalisations by guinea-pig pups,
and wherein said NK-1 receptor antagonist is long acting as determined by its ability to inhibit foot-tapping in the gerbil with ≤ 25-fold loss of potency where the NK-1 receptor antagonist is administered 24 hours prior to NK-1 receptor agonist challenge compared with the potency when administered immediately prior to the NK-1 receptor agonist challenge, with the proviso that the ID₅₀≤10mg/kg i.v. after 24 hour pre-treatment.

4. A use according to any one of claims 1 to 3 wherein the NK-1 receptor antagonist is:
2-(R)-(1-(R)-(3,5-bis(trifluoromethyl)phenyl)ethoxy)-3-(S)-(4-fluorophenyl)-4-(3-(5-oxo-1H,4H-1,2,4-triazolo)methyl)morpholine;
or a pharmaceutically acceptable salt thereof.

## Patentansprüche

1. Verwendung eines NK-1-Rezeptor-Antagonisten zur Herstellung eines Medikaments für die orale Verabreichung zur Behandlung oder Verhütung einer Streßerkrankung, ausgewählt aus akuter Streßerkrankung, Anpassungsstörung und akuter, chronischer oder verzögert einsetzender posttraumatischer Streßerkrankung, ohne gleichzeitige Behandlung mit anderen Antistreßmitteln,
wobei der NK-1-Rezeptor-Antagonist in das ZNS gelangt, wie festgestellt durch sein Vermögen zur Inhibierung des durch einen NK-1-Rezeptor-Agonisten induzierten Fußklopfens bei der Wüstenrennmaus, und bei der Abschwächung von trennungsbedingten Lautäußerungen von Meeerschweinchenjungen wirksam ist,
und wobei der NK-1-Rezeptor-Antagonist langwirkend ist, wie festgestellt durch sein Vermögen zur Inhibierung des Fußklopfens bei der Wüstenrennmaus mit einem ≤ 25fachen Wirksamkeitsverlust, wenn der NK-1-Rezeptor-Antagonist 24 Stunden vor der Herausforderung mit einem NK-1-Rezeptor-Agonisten verabreicht wird, im Vergleich zu der Wirksamkeit, wenn er unmittelbar vor der Herausforderung mit einem NK-1-Rezeptor-Agonisten verabreicht wird, mit der Maßgabe, daß der ID₅₀-Wert nach 24 Stunden Vorbehandlung ≤ 10 mg/kg i.v. beträgt.

2. Verwendung eines NK-1-Rezeptor-Antagonisten zur Herstellung eines Medikaments für die orale Verabreichung zur Behandlung oder Verhütung einer Streßerkrankung, ausgewählt aus akuter Streßerkrankung, Anpassungsstörung und akuter, chronischer oder verzögert einsetzender posttraumatischer Streßerkrankung, ohne gleichzeitige Behandlung mit anderen Antistreßmitteln bei einem Patienten, der nicht auf Benzodiazepine anspricht oder bei dem Benzodiazepine kontraindiziert sind,
wobei der NK-1-Rezeptor-Antagonist in das ZNS gelangt, wie festgestellt durch sein Vermögen zur Inhibierung des durch einen NK-1-Rezeptor-Agonisten induzierten Fußklopfens bei der Wüstenrennmaus, und bei der Abschwächung von trennungsbedingten Lautäußerungen von Meeerschweinchenjungen wirksam ist,
und wobei der NK-1-Rezeptor-Antagonist langwirkend ist, wie festgestellt durch sein Vermögen zur Inhibierung des Fußklopfens bei der Wüstenrennmaus mit einem ≤ 25fachen Wirksamkeitsverlust, wenn der NK-1-Rezeptor-Antagonist 24 Stunden vor der Herausforderung mit einem NK-1-Rezeptor-Agonisten verabreicht wird, im Vergleich zu der Wirksamkeit, wenn er unmittelbar vor der Herausforderung mit einem NK-1-Rezeptor-Agonisten verabreicht wird, mit der Maßgabe, daß der ID₅₀-Wert nach 24 Stunden Vorbehandlung ≤ 10 mg/kg i.v. beträgt.

3. Verwendung eines NK-1-Rezeptor-Antagonisten zur Herstellung eines Medikaments für die orale Verabreichung zur Behandlung oder Verhütung einer Streßerkrankung, ausgewählt aus akuter Streßerkrankung, Anpassungsstörung und akuter, chronischer oder verzögert einsetzender posttraumatischer Streßerkrankung, ohne gleichzeitige Behandlung mit anderen Antistreßmitteln bei einem Patienten, der nicht auf heterocyclische Antidepressiva oder SSRIs anspricht oder bei dem heterocyclische Antidepressiva oder SSRIs kontraindiziert sind,
wobei der NK-1-Rezeptor-Antagonist in das ZNS gelangt, wie festgestellt durch sein Vermögen zur Inhibierung des durch einen NK-1-Rezeptor-Agonisten induzierten Fußklopfens bei der Wüstenrennmaus, und bei der Abschwächung von trennungsbedingten Lautäußerungen von Meeerschweinchenjungen wirksam ist,
und wobei der NK-1-Rezeptor-Antagonist langwirkend ist, wie festgestellt durch sein Vermögen zur Inhibierung des Fußklopfens bei der Wüstenrennmaus mit einem ≤ 25fachen Wirksamkeitsverlust, wenn der NK-1-Rezeptor-Antagonist 24 Stunden vor der Herausforderung mit einem NK-1-Rezeptor-Agonisten verabreicht wird, im Vergleich zu der Wirksamkeit, wenn er unmittelbar vor der Herausforderung mit einem NK-1-Rezeptor-Agonisten verabreicht wird, mit der Maßgabe, daß der ID₅₀-Wert nach 24 Stunden Vorbehandlung ≤ 10 mg/kg i.v. beträgt.

4. Verwendung nach irgendeinem der Ansprüche 1 bis 3, wobei der NK-1-Rezeptor-Antagonist 2-(R)-(1-(R)-(3,5-Bis(trifluormethyl)phenyl)ethoxy)-3-(S)-4-fluorphenyl)-4-(3-(5-oxo-1H,4H-1,2,4-triazolo)methyl)morpholin oder ein pharmazeutisch annehmbares Salz davon ist.

## Revendications

1. Utilisation d'un antagoniste du récepteur NK-1 pour la fabrication d'un médicament pour administration orale pour le traitement ou la prévention d'un état de stress choisi parmi l'état de stress aigu, le trouble d'adaptation et l'état de stress post-traumatique aigu, chronique ou à début retardé, sans traitement concomitant avec d'autres agents anti-stress,
dans laquelle ledit antagoniste du récepteur NK-1 est un médicament pénétrant dans le SNC comme déterminé par sa capacité à inhiber le trépignement provoqué par l'agoniste du récepteur NK-1 chez la gerbille et atténue efficacement Les vocalisations provoquées par la séparation chez le petit du cochon d'Inde,
et dans laquelle ledit antagoniste du récepteur NK-1 a une action prolongée comme le montre sa capacité à inhiber le trépignement chez la gerbille avec une perte de puissance, lorsque l'antagoniste du récepteur NK-1 est administré 24 heures avant l'exposition à l'agoniste du récepteur NK-1, inférieure ou égale à 25 fois la puissance lors de l'administration immédiatement avant l'exposition à l'agoniste du récepteur NK-1, à la condition que la DI₅₀ ≤ 10 mg/kg IV après prétraitement de 24 heures.

2. Utilisation d'un antagoniste du récepteur NK-1 pour la fabrication d'un médicament pour administration orale pour le traitement ou la prévention d'un état de stress choisi parmi l'état de stress aigu, le trouble d'adaptation et l'état de stress post-traumatique aigu, chronique ou à début retardé, sans traitement concomitant avec d'autres agents anti-stress, chez un patient ne répondant pas aux benzodiazépines ou pour lequel les benzodiazépines sont contre-indiqués,
dans laquelle ledit antagoniste du récepteur NK-1 est un médicament pénétrant dans le SNC comme déterminé par sa capacité à inhiber le trépignement provoqué par l'agoniste du récepteur NK-1 chez la gerbille et atténue efficacement les vocalisations provoquées par la séparation chez le petit du cochon d'Inde,
et dans laquelle ledit antagoniste du récepteur NK-1 a une action prolongée comme le montre sa capacité à inhiber le trépignement chez la gerbille avec une perte de puissance, lorsque l'antagoniste du récepteur NK-1 est administré 24 heures avant l'exposition à l'agoniste du récepteur NK-1, inférieure ou égale à 25 fois la puissance lors de l'administration immédiatement avant l'exposition à l'agoniste du récepteur NK-1, à la condition que la DI₅₀ ≤ 10 mg/kg IV après prétraitement de 24 heures.

3. Utilisation d'un antagoniste du récepteur NK-1 pour la fabrication d'un médicament pour administration orale pour le traitement ou la prévention d'un état de stress choisi parmi l'état de stress aigu, le trouble d'adaptation et l'état de stress post-traumatique aigu, chronique ou à début retardé, sans traitement concomitant avec d'autres agents anti-stress, chez un patient ne répondant pas aux anti-dépresseurs hétérocycliques ou ISRS ou pour lequel les ISRS sont contre-indiqués,
dans laquelle ledit antagoniste du récepteur NK-1 est un médicament pénétrant dans le SNC comme déterminé par sa capacité à inhiber le trépignement provoqué par l'agoniste du récepteur NK-1 chez la gerbille et atténue efficacement Les vocalisations provoquées par la séparation chez le petit du cochon d'Inde,
et dans laquelle ledit antagoniste du récepteur NK-1 a une action prolongée comme le montre sa capacité à inhiber le trépignement chez la gerbille avec une perte de puissance, lorsque l'antagoniste du récepteur NK-1 est administré 24 heures avant l'exposition à l'agoniste du récepteur NK-1, inférieure ou égale à 25 fois la puissance lors de l'administration immédiatement avant l'exposition à l'agoniste du récepteur NK-1, à la condition que la DI₅₀ ≤ 10 mg/kg IV après prétraitement de 24 heures.

4. Utilisation selon l'une quelconque des revendications 1 à 3 dans laquelle l'antagoniste du récepteur NK-1 est :
le 2-(R)-(1-(R)-(3,5-bis(trifluorométhyl)phényl)éthoxy)-3-(S)-(4-fluorophényl-4-(3-(5-oxo-1H,4H-1,2,4-triazolo)méthyl)morpholine)
ou un de ses sels pharmaceutiquement acceptable.
